(19)

Europäisches Patentamt

European Patent Office

Office européen des brevets

(11)  **EP 3 490 712 B1**

(12)  **EUROPEAN PATENT SPECIFICATION**

(45) Date of publication and mention
of the grant of the patent:
**09.02.2022  Bulletin 2022/06**

(21) Application number: **17734536.0**

(22) Date of filing: **28.04.2017**

(51) International Patent Classification (IPC):
**B01L 3/00** (2006.01)   **A61M 1/36** (2006.01)
**G01N 15/10** (2006.01)   **B03B 1/04** (2006.01)

(52) Cooperative Patent Classification (CPC):
**B01L 3/502753; A61M 1/3603; A61M 1/3616;**
**A61M 1/362; A61M 1/3678;** A61M 2205/0244;
B01L 3/502761; B01L 2200/0652; B01L 2400/0436;
B03B 1/04; G01N 2015/1081

(86) International application number:
**PCT/US2017/030232**

(87) International publication number:
**WO 2018/022158 (01.02.2018 Gazette 2018/05)**

(54)  **ACOUSTIC SEPARATION FOR BIOPROCESSING**

AKUSTISCHE TRENNUNG ZUR BIOVERARBEITUNG

SÉPARATION ACOUSTIQUE POUR BIOPROCÉDÉS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **28.07.2016  US 201662367773 P**

(43) Date of publication of application:
**05.06.2019  Bulletin 2019/23**

(73) Proprietor: **The Charles Stark Draper Laboratory,**
**Inc.**
**Cambridge, MA 02139 (US)**

(72) Inventors:
• **FIERING, Jason, O.**
**Boston, MA 02210 (US)**
• **KOTZ, Kenneth**
**Newton, MA 02465 (US)**

(74) Representative: **FRKelly**
**27 Clyde Road**
**Dublin D04 F838 (IE)**

(56) References cited:
**WO-A1-2014/046605   WO-A1-2016/062975**

**WO-A2-2007/128795   US-A1- 2015 017 678**

• **ANDREAS LENSHOF ET AL: "Efficient**
**Purification of CD4+ Lymphocytes from**
**Peripheral Blood Progenitor Cell Products Using**
**Affinity Bead Acoustophoresis", CYTOMETRY**
**PART A, vol. 85, no. 11, 22 July 2014 (2014-07-22) ,**
**pages 933-941, XP055404148, DOI:**
**10.1002/cyto.a.22507**
• **DYKESJ ET AL: "Efficient Removal of Platelets**
**from Peripheral Blood Progenitor Cell Products**
**Using a Novel Micro-Chip Based**
**Acoustophoretic Platform", PLOS ONE, PUBLIC**
**LIBRARY OF SCIENCE, US , vol. 6, no. 8 9 August**
**2011 (2011-08-09), pages E23074-1,**
**XP003030948, ISSN: 1932-6203, DOI:**
**10.1371/JOURNAL.PONE.0023074 Retrieved**
**from the Internet:**
**URL:http://www.plosone.org/article/info%3A**
**doi%2F10.1371%2Fjournal.pone.0023074**
**[retrieved on 2011-08-09]**
• **YE AI ET AL: "Separation of Escherichia coli**
**Bacteria from Peripheral Blood Mononuclear**
**Cells Using Standing Surface Acoustic Waves",**
**ANALYTICAL CHEMISTRY, vol. 85, no. 19, 23**
**August 2013 (2013-08-23), pages 9126-9134,**
**XP055261589, US ISSN: 0003-2700, DOI:**
**10.1021/ac4017715**

EP 3 490 712 B1

**Description**

FIELD OF TECHNOLOGY

The present invention relates to an in vitro method of separating target cells from non-target cells. In particular, the present invention relates to an in vitro method of separating target cells from non-target cells in a biofluid.

SUMMARY

The present invention relates to an in vitro method of separating target cells from non-target cells in a biofluid. The method may comprise pretreating the biofluid, flowing the pretreated biofluid into an inlet of a microfluidic separation channel, and applying acoustic energy to the microfluidic separation channel to accumulate target cells within a primary stream along the separation channel and accumulate non-target cells within a secondary stream along the separation channel. In some embodiments, pretreating the biofluid comprises introducing a predetermined volume of at least one additive into the biofluid to alter at least one of size of the target cells, size of the non-target cells, compressibility of the biofluid, compressibility of the target cells, compressibility of the non-target cells, and aggregation potential of the non-target cells; and altering at least one of density of the biofluid, density of the target cells, and density of the non-target cells to produce a pretreated biofluid, the predetermined volume of the at least one additive being selected to alter or regulate the biofluid to have a desired density. In some embodiments, the acoustic energy may be applied to the pre-treated biofluid within the microfluidic separation channel to separate the target cells from the non-target cells.

In some embodiments, the method further comprises selecting the biofluid from blood buffy coat, leukapheresis product, peripheral blood, whole blood, lymph fluid, synovial fluid, spinal fluid, bone marrow, ascities fluid, and combinations or subcomponents thereof.

According to some embodiments, the method further comprises selecting the target cells to be leukocytes selected from the group consisting of mononuclear cells, lymphocytes, monocytes, granulocytes, agranulocytes, macrophages, T cells, B cells, NK cells, subclasses thereof, and combinations thereof. Thus, in accordance with one embodiment, there is provided a method of separating leukocytes from non-target cells. According to certain embodiments, there is provided a method of separating lymphocytes from non-target cells. The method may comprise selecting the target cells to be stem cells.

The biofluid may be collected from a donor subject. The primary stream may be post-treated and delivered to a recipient subject. The recipient subject may be the same as the donor subject. The method may be performed in line such that the biofluid is collected from a subject, target cells are separated from non-target cells in the biofluid by a method as described herein, a fluid enriched in target cells may be post-treated, and the post-treated fluid may be delivered back to the subject. The donor subject and the recipient subject may not the same subject. The fluid enriched in target cells may be collected and stored for delivery to the recipient subject at a later time.

The method may further comprise flowing the fluid comprising the target cells through microfluidic separation channels arranged in series and applying acoustic energy to each separation channel. The biofluid comprising target cells may be flowed through a first microfluidic separation channel to produce a primary stream enriched in target cells. The primary stream may then be flowed through a second microfluidic separation channel to produce a second primary stream having a higher degree of enrichment of target cells.

For instance, the method may further comprise dosing the at least one primary stream with a reagent to produce a dosed suspension. The reagent may be selected from an antigen or activation reagent configured to biochemically induce cell activation. The dosed suspension may be flowed through a second microfluidic separation channel to produce a primary stream enriched in activated cells.

For instance, in some embodiments of the method, the target cells in the primary stream may be lymphocytes and the method may further comprise separating activated lymphocytes from non-activated lymphocytes in the primary stream. The method may further comprise dosing the lymphocyte primary stream with a reagent to produce the dosed suspension, flowing the dosed suspension into an inlet of a second microfluidic separation channel, and applying acoustic energy to the second microfluidic separation channel. Activated lymphocytes may accumulate within at least one primary stream along the second separation channel and non-activated lymphocytes may accumulate within at least one secondary stream along the second separation channel.

According to certain embodiments, the method further comprises flowing a second fluid adjacent to the biofluid into an inlet of the microfluidic separation channel. The second fluid may be introduced into the microfluidic separation channel through a separate inlet. The biofluid and the second fluid may flow through the separation channel in substantially parallel and substantially laminar form.

At least one of the target cells and the non-target cells may be live cells, frozen cells, preserved cells, or cells grown in a cell culture. The microfluidic separation channel may be formed of a thermoplastic material. The microfluidic separation channel may be disposable.

BRIEF DESCRIPTION OF THE DRAWINGS

The accompanying drawings are not intended to be drawn to scale. In the drawings, each identical or nearly identical component that is illustrated in various figures is represented by a like numeral. For purposes of clarity, not every component may be labeled in every drawing. In the drawings:

FIG. 1 is a schematic drawing of a microfluidic separation channel, according to one embodiment;

FIG. 2 is a schematic drawing of an alternate microfluidic separation channel, according to another embodiment;

FIG. 3 is a micrograph of a microfluidic separation channel coupled to an acoustic transducer that is turned off;

FIG. 4 is a micrograph of a microfluidic separation channel coupled to an acoustic transducer that is turned on;

FIG. 5 is a schematic drawing of a system for microfluidic cell separation;

FIG. 6 is a schematic drawing of an alternate system for microfluidic cell separation,;

FIG. 7 is a graph of the percentage of recovery for lymphocytes and other white blood cells from leukapheresis fluid, buffy coat, and whole blood after separation according to one embodiment of a method of separating lymphocytes and white blood cells from other cells in a biofluid;

FIG. 8 is a graph of lymphocyte purity as a percentage of all white blood cells from leukapheresis fluid, buffy coat, and whole blood after separation according to one embodiment of a method for separating lymphocytes from other cells in a biofluid;

FIG. 9 is a graph of the relative population of lymphocytes among all white blood cells in a fraction collected after separation according to one embodiment of a method of separating cells in a biofluid;

FIG. 10 is a graph of the separation ratio of lymphocytes to erythrocytes, leukocytes, or a side stream in a fraction collected after separation according to one embodiment of a method of separation cells in a biofluid;

FIG. 11 is a graph of the separation ratio of lymphocytes to erythrocytes, leukocytes, or a side stream in a fraction collected after separation according to an alternate embodiment of a method of separation cells in a biofluid;

FIG. 12 is a graph comparing lymphocyte to monocyte separation ratio in the fraction collected after separation, according to one embodiment of a method of separating cells in a biofluid;

FIG. 13 is a comparison graph of lymphocyte separation ratio and erythrocyte removal from samples pretreated with a cell aggregator as compared to samples pretreated with a density gradient medium, according to one embodiment of a method of separating cells in a biofluid; and

FIG. 14 is a schematic drawing of an alternate microfluidic separation channel, according to another embodiment.

## DETAILED DESCRIPTION

In the fields of cell therapy and bioprocessing emerging medical techniques may involve extraction of blood or tissue from a patient followed by purification of a particular cell type from a sample. In some applications, the particular cell type is prepared for treatment or manipulated before it is re-injected into a patient.

WO2016/062975 relates to a method of treating a biological sample, preferably a sample of blood or bodily fluids likely to contain one or more species of interest, and comprising a step of decomplexification by acoustopherisis. WO2016/062975 also relates to associated systems, devices, substrates and connection devices.. Dykes *et al.* 2011 relates to a microchip-based acoustophoresis technique, utilizing ultrasonic standing wave forces for the removal of platelets from peripheral blood progenitor cell products. Yi *et al.* 2013 relates to a microfluidic device that was developed to separate heterogeneous particle or cell mixtures in a continuous flow using acoustophoresis.

Aspects and embodiments disclosed herein relate to separating target cells from non-target cells in a biofluid. In particular, one example application is the separation of leukocytes or a subclass of leukocytes from a blood sample.

Disclosed herein are methods and systems for use in processing of cells for cell therapy. Many other uses could also be envisioned, in particular wherever a particular cell type is desired to be collected from a cell suspension or natural sample. Some non-limiting examples include the diagnostic or environmental monitoring assays, tissue engineering, *in vitro* models, and biomanufacturing systems, such as for energy applications.

Formerly cell selection for applications within bioprocessing has been performed by one or more batch centrifugations, continuous centrifugations, magnetic separation, or combinations thereof. Centrifugation is only able to separate particles by density, limiting its ability to separate leukocyte subclasses from other types of leukocytes. Addition of a density medium may improve leukocyte stratification, but only in small batch procedures requiring technically trained operators. Furthermore, no form of centrifugation is able to separate subclasses of lymphocytes such as T cells from B cells.

Magnetic separation can be highly selective, but depends on the attachment of paramagnetic capture particles to cells using affinity ligands, such as antibodies. The particles may pose a safety risk if injected into a patient. Accordingly, the magnetic particles must be removed from a final therapeutic product. The efficiency of magnetic separation varies with the load of interfering cells or with the concentration of background proteins contained in the sample that may specifically or non-specifically bind to the affinity ligand. Additionally, the attachment of certain magnetic particles to cells through affinity ligands may be irreversible. In this case, only one separation can be done using a general magnetic force. For these reasons, magnetic separation is a complex procedure that is usually insufficient for bioprocessing workflow.

Aspects and embodiments disclosed herein may be advantageous over previous cell separation technologies because, for example, the purification of desired cells can be performed continuously, in some embodiments, the method of the invention provides separation by both size and density to further enhance cell separation, the separation processes may be readily scaled to small or large sample volumes, a high degree of purification can be achieved without the use of antibodies, ligands, immunochemistry, or other foreign particles, and further purification can be achieved with the addition of safely injectable, physiologically acceptable additives.

One non-limiting example of cell therapy that may be performed using the method described herein is CAR-T therapy for the treatment of blood cancers. The therapy may involve engineering chimeric antigen receptors on T-cells by viral transduction or other gene editing methods known to those skilled in the art. In CAR-T treatment, blood is generally collected from a patient. The blood may be whole blood, or leukapheresis product. Leukapheresis product is a collection of mainly leukocytes and platelets, with a reduced concentration of erythrocytes, as compared to whole blood. From this collected blood sample, specific subclasses of the leukocytes may be selected for further processing. In CAR-T therapy, the desired classes of cells may vary, but generally include mononuclear cells, lymphocytes, T cells, or subclasses of T cells, such as CD4+, or CD8+. The selected cells may then be modified (transduced) by genetic engineering to enhance their ability to attack malignant cells. The genetic engineering may include incubating to increase their abundance, washing or purifying, testing for quality control, and optionally infusing into a patient.

The aspects and embodiments disclosed herein may improve methods for selecting the desired cells, and may also have applications in other steps in the process such as washing or the cells, or purification of samples after transduction.

Acoustic separation, also referred to as acoustophoresis, may be used to isolate or enrich desired cells as part of a bioprocessing workflow. Acoustic separation of particles in a biofluid has been described in, for example, U.S. Patent Application Publication Nos. 2016-0030660, 2016-0008532, and 2013-0048565, and in U.S. Patent No. 9,504,780. The aspects and embodiments disclosed herein provide separation of target cells from non-target cells in a biofluid. More specifically, the aspects and embodiments disclosed herein provide selective separation between cell types, without requiring the use of an affinity based capture particle.

In acoustic separation, a mixed suspension may flow through a duct that is oscillated at ultrasonic frequencies by an external mechanical oscillator. The duct may form a resonant cavity, for instance so that ultrasonic pressure waves are generated and contact the flow across the duct. For example, the ultrasonic waves may be generated at an angle relative to the flow. Ultrasonic waves may be generated in a direction substantially transverse to the flow. Cells or other particles in the suspension may experience a force from the pressure waves and migrate to nodes in the resulting pressure field. The rate at which the cells migrate generally depends on particle size, density, and compressibility. Separation may be facilitated, for example, by larger and more dense cells migrating to a pressure node, with smaller or neutrally buoyant cells migrating slowly, not migrating (substantially staying on axis), or migrating to anti-nodes. For instance, in a typical configuration separation process, the pressure node is established along the axis of the duct and certain particles may move to this pressure node axis and flow in a concentrated stream along it, while other cells may remain disperse or move to a pressure anti-node axis.

Referring again to the example application of CAR-T therapy, lymphocytes may be preferentially extracted from blood samples. The therapy may involve altering a property of the cell suspension or of a certain class of cells within the suspension, such that lymphocytes are less susceptible to acoustic energy than, for example erythrocytes and other classes of leukocytes. Therefore when a cell suspension, for example a blood sample, is passed through an acoustic separator, lymphocytes may remain in a side stream with greater abundance than undesired cells. The side stream may be collected for processing and the center stream may be discarded.

In accordance with an aspect, there is provided a method of separating target cells from non-target cells in a biofluid. More specifically, there is provided a method for selective, differential separation of a desired cell type from a biofluid comprising a suspension of mixed cell types. Target cells which may be selectively separated from the mixed cell types in the suspension include leukocytes, mononuclear cells, lymphocytes, monocytes, granulocytes, agranulocytes, macrophages, T cells, B cells, NK cells, subclasses thereof, and combinations thereof. For instance, in some embodiments, target cells are subclasses of T cells, including but not limited to CD4+, CD8+, $T_H$, $T_{CM}$, and $T_{FH}$ cells. In some embodiments, target cells are selected to be stem cells.

Non-target cells may comprise any and all cells not selected as the target cell. Non-target cells may comprise erythrocytes, platelets, granulocytes, monocytes, macrophages, leukemic cells, and leukocytes excluding the leukocytes selected as target cells. In some embodiments, the non-target cells are platelets and erythrocytes. Erythrocytes are approximately the same size as lymphocytes. In order to separate erythrocytes from lymphocytes in a biofluid, efficiency may be greatly increased by including an additive to alter or regulate at least one parameter of the biofluid. For instance, the additive may alter the aggregation potential of non-target cells and/or the density of the biofluid. According to certain embodiments, the additive is introduced in sufficient volume to regulate the density of the biofluid to be substantially similar to the density of the lymphocytes.

According to certain embodiments, target cells are separated from non-target cells to produce a target cell enriched fluid. The target cells and/or non-target cells may be live cells, frozen cells, preserved cells, or cells grown in a cell culture. The target cell enriched fluid may comprise a higher concentration of target cells, as compared to the input biofluid or the pretreated biofluid.

Generally, a biofluid, for example whole blood, comprises a high concentration of erythrocytes. To produce a target cell enriched fluid, it may be desirable to selectively deplete erythrocytes.

The method of separating target cells from non-target cells in a biofluid may further comprise providing a biofluid. The biofluid may be obtained from a donor subject. The donor subject's biofluid may be subjected to down-stream processes directly, or may be collected and stored for later processing. As used herein, "directly" refers to processing of the biofluid without subjecting the biofluid to a long-term storage period. For instance, the biofluid may be processed immediately in an in-line arrangement, within minutes, or within hours. The biofluid may be stored for one day or more. The biofluid is collected from a donor subject through an intraluminal line. Accordingly, the method may further comprise obtaining the biofluid from a donor subject through an intraluminal line. As used herein, an "intraluminal" line refers to a transfusion line connectable to a lumen of a subject. More specifically, an intraluminal line may be connectable to a body cavity, tubular structure, or organ in the body, such as a vein, an artery, the bladder, or intestine. For instance, a transfusion line may be connectable to the circulatory or gastrointestinal system of the subject. The intraluminal line includes, for example, intravenous lines, central venous lines, intravascular lines, intratissue lines, catheters, and transfusion lines. The intraluminal line catheter may be, for example, a peripheral indwelling catheter, an intravenous catheter, or a central venous catheter.

As used herein, the term "subject" is intended to include human and non-human animals, for example, vertebrates, large animals, and primates. In certain embodiments, the subject is a mammalian subject, and in particular embodiments, the subject is a human subject. Although applications with humans are clearly foreseen, veterinary applications, for example, with non-human animals, are also envisaged herein. The term "non-human animals" of the invention includes all vertebrates, for example, non-mammals (such as birds, for example, chickens; amphibians; reptiles) and mammals, such as non-human primates, domesticated, and agriculturally useful animals, for example, sheep, dog, cat, cow, pig, rat, among others.

The biofluid may be obtained from a standard blood processing device. For instance, the biofluid may be obtained from an apharesis machine. The biofluid may be directly obtained from a standard blood processing device and further processed immediately, for example in an in-line arrangement. The biofluid may be obtained from a standard blood processing device and stored for one day or more before being introduced into the microfluidic separation chamber.

In some embodiments, the method further comprises selecting the biofluid from blood buffy coat, leukapheresis product, peripheral blood, whole blood, lymph fluid, synovial fluid, spinal fluid, bone marrow, ascities fluid, and combinations or subcomponents thereof. The biofluid may comprise a synethetic medium comprising a cell suspension. For instance, the biofluid may comprise a cell culture medium. The biofluid may comprise a subcomponent of a biofluid. For instance, the biofluid may comprise cell enriched biofluid, cell depleted biofluid, diluted biofluid, concentrated biofluid, filtered biofluid, purified biofluid, or otherwise treated biofluid.

As used herein, leukapheresis product refers to a blood product which has undergone an apheresis separation process. The apheresis separation process may have been performed to delepete or enrich for leukocytes. Thus, the leukapheresis product may comprise leukocyte enriched apheresis product or leukocyte depleted apheresis product. The leukapheresis product may comprise synthetic biofluid. The leukapheresis product may be purchased from a manufacturer. In some non-limiting embodiments, the leukapheresis product is LeukoPak™ leukapheresis product, as distributed by AllCells (Alameda, CA).

The method of separating target cells from non-target cells in a biofluid may further comprise pretreating the biofluid. In some embodiments, pretreating the biofluid comprises introducing a predetermined volume of at least one additive into the biofluid to alter at least one of size of the target cells, size of the non-target cells, compressibility of the biofluid, compressibility of the target cells, compressibility of the non-target cells, and aggregation potential of the non-target cells and altering at least one of density of the biofluid, density of the target cells, and density of the non-target cells. The additive may be cell-friendly. For instance, in some embodiments, the concentration of additive introduced into the biofluid is generally safe for intraluminal injection into a subject. In some embodiments, the additive selected is physiologically acceptable and generally safe for intraluminal injection into a subject.

Generally, the method may comprise introducing an additive to modify the biofluid or cell chemistry, to enhance separation of target cells from non-target cells. For instance, the biofluid's electrolyte concentration (i.e. salinity or tonicity) may be adjusted, such that a desired cell type is enlarged, swollen, crenated, sphered, or rigidified in response. The desired cell type may be the target cell or the non-target cell. The change in one or more physical properties of the cell type may affect the response of the cell to the applied acoustic force within the microfluidic separation channel, enabling a differential separation between the desired cell type and other cell types within the biofluid. The method may comprise selecting the additive from the group consisting of a cell aggregator, deionized water, a detergent, a surfactant, a solution to regulate salinity of the biofluid, a solution to regulate tonicity of the biofluid, a solution to regulate viscosity of the biofluid, a solution to regulate osmolarity of the biofluid, a solution to regulate ion concentration of the biofluid, and combinations thereof.

The method may comprise introducing an additive to alter size or shape of the target cells or non-target cells. As previously mentioned, a desired cell type may become swollen, crenated, sphered, or rigidified in response to the introduction of an additive in the biofluid. The change in size or shape may facilitate discrimination between the cell types in the separation process. An additive may also be introduced to activate a desired cell type, whereby an activated T cell may be larger than a non-activated T cell. Thus, natural morphological changes due to biochemically induced activation or the natural cell cycle may be exploited to separate target cells from non-target cells.

The method may comprise introducing an additive to alter sodium or ion concentration of the biofluid. For instance, a concentrated sodium chloride solution may be introduced to crenate and/or shrink erythrocytes and other non-target cells by osmosis. Without wishing to be bound by a particular theory, it is believed that hemoglobin contained within erythrocytes will effectuate an increase in density simultaneously with a decrease in volume of the cell. Thus, it may be possible to selectively increase the density of erythrocytes by decreasing their size, to promote an enhanced separation of target cells from non-target erythrocytes.

In some embodiments, the method comprises introducing an additive to alter compressibility of the biofluid, target cells, or non-target cells. For instance, detergents and/or surfactants may be added to alter cell membrane mechanics, such that desired cell types undergo a change in compressibility. In some embodiments, detergents or surfactants alter the cell membrane, such that desired cell types are more susceptible to changes in ion concentration in the biofluid. For instance, non-ionic detergents may comprise the Tween™ family of detergents, Brij-35™ detergents, or the Pluronic™ family of detergents. Such detergents are known to affect membrane permeability and cellular biomechanics at very low concentrations of less than 0.05% weight/volume fraction of detergent. Thus, by adding certain detergents, it may be possible to differentially affect the cellular mechanical properties of cell density, shape, size or ionic content in order to promote an enhanced separation of target cells from non-target cells.

An additive may be introduced into the biofluid to alter aggregation potential of the target cells or the non-target cells. As used herein, "aggregation potential" refers to the mechanism by which a desired cell type aggregates, agglutinates, adheres, or forms a complex with like cells. In some embodiments, the aggregation potential refers to a desired cell type's ability to aggregate with cells of a different cell type. For instance, an additive may be introduced to alter or regulate the aggregation potential of erythrocytes or platelets. Generally, many biofluids comprise a high concentration of erythrocytes and/or platelets. By aggregating the erythrocytes and/or platelets, a more efficient separation from other cells may be achieved.

In some embodiments, the aggregation potential is altered or regulated by an additive that prohibits a desired cell type from binding, aggregating, agglutinating, adhering, or forming a complex with a like or different cell type. For instance, the aggregation potential may be altered or reduced by an anti-coagulant. In other embodiments, the aggregation potential may be altered, enhanced, or regulated by a cell aggregator. As used herein, a "cell aggregator" refers to an additive that may bind, aggregate, adhere, agglutinate or form a complex with a desired cell type. A "cell aggregator" may also refer to an additive that may cause a desired cell type to bind, aggregate, adhere, agglutinate, or form a complex with like or different cell types. The cell aggregator may cause cells to aggregate by activating natural biochemical pathways, by altering cell mechanics, or by reducing or screening electrostatic barriers between cells in the pre-treated biofluid.

In some embodiments, the method further comprises selecting the cell aggregator to be a long-chain polysaccharide. Long-chain polysaccharides include, but are not limited to, dextran, polysucrose, hetastarch (hydroxyethyl starch), and Ficoll™ media, distributed by GE Healthcare (Chicago, IL). The long-chain polysaccharide may have a molecular weight between about 100 kD and about 500 kD. In some embodiments, the long-chain polysaccharide has a molecular weight between about 250kD and about 500 kD, between about 200 kD and about 400 kD, between about 300 kD and about 400 kD. The long-chain polysaccharide may have a molecular weight of about 100 kD, about 200 kD, about 250 kD, about 300 kD, about 400 kD, and about 500 kD. In some embodiments, the cell aggregator comprises a long-chain polysaccharide present at a concentration of between about 0.5% (w/v) and about 25% (w/v). In some embodiments, the cell aggregator comprises a long-chain polysaccharide present at a concentration of between about 1.0% (w/v) and about 20% (w/v), between about 5.0% (w/v) and about 15% (w/v), between about 8.0% (w/v) and about 12% (w/v). For instance, the cell aggregator may comprise a long-chain polysaccharide present at about 0.5% (w/v), about 1.0% (w/v), about 2.0% (w/v), about 5.0% (w/v), about 8.0% (w/v), about 10% (w/v), about 12% (w/v), about 15% (w/v), about 20% (w/v), about 24% (w/v), and about 25% (w/v).

In some embodiments, the method further comprises selecting the cell aggregator to be a platelet aggregator or a cell adhesion molecule (CAM). The CAM may be released or obtainable from an activated platelet granule. Such CAMs aggregate platelets by known natural mechanisms. Platelet activation may induce the platelet to releases granules and exposed the contents of platelet granules on the outside of the cell. CAMs may then promote platelet aggregation through platelet-fibrin and platelet-platelet binding. CAMs may be released from an activated platelet granule by biochemically inducing their release, for example through activation by addition of thrombin, Type II collagen or adenosine diphosphate, or by introducing natural or synthetic CAMs obtained from a distributor into the biofluid. The CAMs released or obtainable from an activated platelet granule may include, but are not limited to, P-selectin and von Willebrand factor. Platelet activators include, but are not limited to, adenosine diphosphate, thrombin, Type II collagen, and ristocetin.

An additive may be introduced into the biofluid to alter density of the biofluid. In some embodiments, the additive is selected from a density gradient medium, a density additive, and combinations thereof. Density gradient media is a media for cell isolation, generally used in the practice of centrifugal separation. Density gradient media are well known in the art and include, for example, ACCUSPIN™ media, Histodenz™ media, OptiPrep™ media, and Histopaque® media distributed by Sigma-Aldrich (St. Louis, MO), Ficoll-Paque™ media and Percoll™ media distributed by GE Healthcare (Chicago, IL), RosetteSep™ media and Lymphoprep™ media distributed by STEMCELL Technologies (Vancouver, Canada). The list of density gradient media is merely exemplary and non-exhaustive. A density additive may comprise a reagent having a different density than the biofluid, or configured to regulate or alter the density of the biofluid. For instance, the density additive may comprise pure water, deionized water, a salt, a saline buffer solution, or a nonionic iodinated compound. Nonionic iodinated compounds include, but are not limited to, diatrizoic acid, meglumine diatrizoate, and iodixanol. According to certain embodiments, the density additive is selected to be cell-friendly, such that it does not increase osmolarity of the biofluid to a degree that would be harmful to the cells. For instance, the density additive may be selected to not comprise cesium chloride or sucrose.

In some embodiments, the additive is introduced to alter or regulate the density of the biofluid to be within a range of the density of the target cells or non-target cells. For example, the density may be regulated such that target cells approach neutral acoustic buoyancy in the biofluid, reducing the acoustic force acting on them, as compared to the force acting on the non-target cells. The density of the biofluid may be regulated to a density of between about 1.00 g/mL and about 1.15 g/mL. In some embodiments, the density of the biofluid is regulated to a density of between about 1.00 g/mL and about 1.10 g/mL, between about 1.10 g/mL and about 1.15 g/mL, between about 1.02 g/mL and about 1.09 g/mL, between about 1.03 g/mL and about 1.08 g/mL, between about 1.04 g/mL and about 1.07 g/mL, and between about 1.045 g/mL and about 1.065 g/mL. Specifically, the density of the biofluid may be regulated or altered to a density of about 1.00 g/mL, about 1.01 g/mL, about 1.02 g/mL, about 1.03 g/mL, about 1.04 g/mL, about 1.05 g/mL, about 1.06 g/mL, about 1.07 g/mL, about 1.08 g/mL, about 1.09 g/mL, about 1.10 g/mL, about 1.12 g/mL, and about 1.15 g/mL.

Pretreating the biofluid may further comprise introducing an additive to alter density of the target cells or non-target cells. The additive may be introduced to alter or regulate the density of biofluid and cells to be within a range of each other, for instance to make the cells approach neutral acoustic buoyancy within the fluid. A diluent, salt, or saline solution may be introduced to alter or regulate the density of target cells or non-target cells to illicit a certain response from a desired cell type or to have a density within a range of the density of the biofluid. For instance, sodium or an ion concentration may be reduced, for example by dilution with deionized water, to swell erythrocytes by osmosis while lymphocytes use known natural mechanisms to regulate their size, increasing size discrimination between the two cell types. In another non-limiting example, leukemic cells swell more readily than healthy lymphocytes, and the additive may facilitate removal of the leukemic cells.

The method may comprise introducing an additive to alter both density of the biofluid and aggregation potential of the non-target cells. In some embodiments, the combination of a density additive and a cell aggregator produces a synergistic effect, whereby the method produces a more efficient separation of target cells from non-target cells and a higher concentration of target cells in the target cell enriched fluid than would be expected from the combination of both effects. For instance, in a method of separating lymphocytes from other leukocytes and erythrocytes, an additive or a combination of additives may be introduced to alter the density of the biofluid and to aggregate erythrocytes. The density additive may enhance the separation of the target cell, or lymphocytes in this example, from the non-target cells (for example, leukocytes), while the cell aggregator may effectively increase the acoustic scattering radius of the non-target cells to enhanced separation of the non-target cells over the lymphocyte or other target cells. The individual additives, when used separately, may not provide sufficient separation of lymphocytes from non-target cells, but the combination may promote an enhanced effective differential separation of target cells from non-target cells.

The additive may further comprise affinity based capture particles. Generally, the affinity based particles are safe for intraluminal injection into a subject. For instance, the additive may comprise biochemical moieties, such as antibodies, that bind target cells or non-target cells. The cell aggregator may comprise a solution comprising antibodies that bind and aggregate target cells or non-target cells. The antibodies may bind and aggregate a desired cell type. The additive may comprise emulsion droplets, gel particles, or lipid encapsulated oil vesicles. The affinity based capture particle is safe for intraluminal injection. The affinity based capture particle may be engineered to be "anti-focusing" or "positively focusing" by designing it with low density or high density. The low density "anti-focusing" capture particle may experience acoustophoretic forces in the opposite direction as the target cells or non-target cells. The high density "anti-focusing" capture particle may experience migration to the pressure anti-node, while target cells or non-target cells migrate toward the pressure node. An acoustic analog to magnetic separation may comprise "positively focusing" capture particles. For instance, a "positively focusing" capture particle may be used to trap a desired cell type, such that selected cells remain held in the separation channel, while other cells flow through. The held cell type may be released at a later time. In some embodiments, a large capture particle molecule may bind to many points on the surface of a desired cell type, and may alter the acoustophoretic force exhibited on the particle by changing its effective diameter.

The method of separating target cells from non-target cells may further comprise flowing biofluid into an inlet of a microfluidic separation channel. For instance, the method may comprise flowing the pretreated biofluid into the microfluidic separation channel. The biofluid may have a flow rate of between about 0.03 mL/min to about 0.5 mL/min. In some embodiments, the biofluid may have a flow rate through the microfluidic separation channel of between about 0.05 mL/min to about 0.4 mL/min, about 0.1 mL/min to about 0.3 mL/min. The biofluid may have a flow rate through the microfluidic separation channel of about 0.03 mL/min, 0.05 mL/min, 0.08 mL/min, 0.1 mL/min, 0.2 mL/min, 0.3 mL/min, 0.4 mL/min, 0.5 mL/min, or any range therebetween.

The method may further comprise applying acoustic energy to the microfluidic separation channel. The acoustic energy may be applied in the form of an acoustic wave. The acoustic wave may be applied at an angle relative to the flow of fluid through the separation channel. The angle and magnitude of the acoustic wave may be engineered based on size of the device, size of the channel, or flow rate of fluid through the channel. The acoustic energy may be applied in a direction substantially transverse to the biofluid flow through the microfluidic separation channel. The acoustic wave may be a standing acoustic wave. The acoustic energy may be applied to the microfluidic separation channel continuously. The continuous application of acoustic energy may allow for a greater efficiency of separation. The acoustic energy may be applied to the microfluidic separation channel intermittently or on a timed schedule. The intermittent energy application may allow for cells to move freely through the channel if there is a blockage.

The applied acoustic energy may act on the cells and particles within the biofluid to drive them according to size, density, and/or compressibility. In some embodiments, the method may comprise accumulating target cells within a primary stream along the separation channel. In some embodiments, the method may comprise accumulating non-target cells within a secondary stream along the separation channel. The accumulation of a cell type within a desired stream along the separation channel may be engineered by adjusting parameters such as wavelength, frequency, amplitude, power level, or other modulation of the applied acoustic energy.

Depending on the target cells or non-target cells selected according to the method, one class of cells may accumulate in response to a pressure node or anti-node generated by the acoustic energy. For instance, target cells may accumulate within a primary stream in response to a pressure node, and non-target cells may accumulate within a secondary stream in response to a pressure anti-node. Generally, particles, including cells, will be driven by the acoustic energy in response to their contrast factor. Particles may migrate at a rate which is proportional to the magnitude and sign of their contrast factors. Particles with a positive contrast factor may be driven to pressure nodes, while particles with a negative contrast factor may be driven to pressure anti-nodes. Particles with a greater magnitude contrast factor are generally driven at a faster rate than particles with a lesser magnitude contrast factor.

The rate at which cells are driven in response to their acoustic energy generally depends on particle size, density, and compressibility. Briefly, the contrast factor is based on the bulk modulus (K) and density (ρ) of a particle, here of the cells. When suspended in a fluid, the contrast factor (φ) for the cells is calculated with the below equation:

$$\varphi = \frac{5\rho - 2 \cdot 1.02}{2\rho + 1.02} + \frac{2.2}{K}$$

In some embodiments, the method of separating target cells from non-target cells in a biofluid comprises collecting the at least one primary stream comprising the target cells. Generally, the biofluid entering the microfluidic separation channel is a well-mixed primary stream, comprising desegregated target cells and non-target cells. Upon experiencing acoustic energy, target cells and non-target cells may generally accumulate into fractions of the general stream of biofluid. The fraction or fractions of biofluid flowing through the microfluidic separation channel selectively enriched in target cells are defined as the primary stream. There may be more than one fraction of biofluid within the microfluidic separation channel enriched in target cells. For instance, target cells may be driven to a pressure node at the center of the channel in one embodiment, and target cells may be driven to the pressure anti-nodes at the periphery of the channel in an alternate embodiment. The location of pressure nodes and anti-nodes within the channel may be designed by positioning the acoustic energy or by selecting frequency and wavelength of the acoustic waves. The primary stream comprising target cells may be collected for storage, immediate use, transfusion into a patient, or for research. Where the method is designed to create a target cell depleted fluid, the primary stream comprising target cells may be discarded as waste.

Similarly, in some embodiments, the method of separating target cells from non-target cells comprises collecting the at least one secondary stream comprising non-target cells. The fraction or fractions within the biofluid selectively depleted in target cells, and selectively enriched in non-target cells are defined as the secondary stream. The target cells and non-target cells may have opposing contrast factors. With opposing contrast factors, the target cells and non-target cells may be driven in opposite directions, or one may be driven away from the general stream, for example to the center or the periphery of the channel. The target cells and non-target cells may have contrast factors of a different magnitude, but the same sign. One class of cells may be driven away at a faster rate than the other, defining the primary and secondary streams. The secondary stream may be collected for storage, for further research, or to be discarded as waste. Where the method is designed to deplete a biofluid of the target cells, the secondary stream may be collected for later use or for transfusion into a patient. The method may comprise collecting the primary stream comprising target cells and further comprise separately collecting the at least one secondary stream comprising the non-target cells.

A target cell enriched or target cell depleted fluid may be post-treated and delivered to a recipient subject. For instance, the primary stream may be post-treated and delivered to a recipient subject. Post-treating a fluid may comprise a process such as washing, separating, concentrating, diluting, heating, purifying, or filtering capable of removing toxins, contaminants, or harmful chemical compounds from the fluid. In general, a fluid is post-treated to produce a physiologically acceptable fluid that may be directly delivered to a recipient subject, for example via an intraluminal line as previously described. The post-treated fluid may be stored for delivery to a recipient subject at a later time.

The target cell enriched or target cell depleted fluid is post-treated to produce a therapeutic fluid. Post-treating the fluid may comprise viral transduction, gene transfer, or gene editing of the target cells to produce a therapeutic, physiologically acceptable fluid for delivery to a recipient subject, as previously described.

The recipient subject may be the same as the donor subject. The donor subject and the recipient subject may not be the same. The donor subject and the recipient subject may generally be physiologically compatible.

The method may be performed in line such that the biofluid is collected from a subject and directly pretreated, target cells are separated from non-target cells in the biofluid by a method as described herein to produce a target cell enriched fluid, the fluid enriched in target cells may be post-treated, and the post-treated fluid may be directly delivered back to the subject. In some embodiments, the method is performed essentially as previously discussed, however the target cells are separated from non-target cells to produce a target cell depleted fluid, which may be post-treated and delivered back to the subject.

According to certain embodiments, the method further comprises flowing a second fluid adjacent to the biofluid into an inlet of the microfluidic separation channel. The inlet may be an inlet separate from the biofluid inlet of the microfluidic separation channel. The biofluid and the second fluid may flow through the separation channel in substantially parallel form. For instance, both fluids may flow through the separation channel at opposite peripheries of the channel, the second fluid may flow through both peripheries of the channel, or the second fluid may flow in the center of the channel. The biofluid and the second fluid may flow through the separation channel in substantially laminar form. As used herein, substantially laminar flow includes substantially ordered flow. Laminar flow may have a Reynolds number (Re) less than about 2100. Laminar flow has a Reynolds number (Re) less than about 4000.

The second fluid may be an inert fluid that may comprise water, deionized water, or phosphate buffered saline (PBS). The second fluid may have its density adjusted with a density gradient medium or density additive, independently from the pre-treated biofluid. The applied acoustic energy may drive target or non-target cells from the biofluid into the essentially parallel flowing second fluid initially comprising no cells, such that the second fluid, now comprising selectively separated cells, may exit the microfluidic separation channel through a separate outlet. Where the target cells are driven into the second fluid, the second fluid comprising target cells is essentially the primary stream. Conversely, where the non-target cells are driven into the second fluid, the second fluid is essentially the secondary stream.

The methods described herein may be performed in a staged separation or in series. Specifically a target cell enriched fluid or a target cell depleted fluid may be further processed by pretreating with an additive, flowing through a second microfluidic separation channel, and applying acoustic energy. The additive introduced into the fluid in the downstream operation may be the same or a different additive as the one introduced into the biofluid in the first pass separation process. Additionally, the target cells selected in the first pass process may be the same or different as those selected in the second pass process. As a non-limiting example, a biofluid may be pretreated and flowed through a microfluidic separation channel to produce a platelet depleted fluid. The output platelet depleted fluid may further be flowed through a second microfluidic separation channel to remove neutrophils and/or monocytes. As another non-limiting example a biofluid may be flowed through a microfluidic separation channel to produce lymphocyte enriched fluid. The lymphocyte enriched fluid may be flowed through a second microfluidic separation channel to produce a further lymphocyte enriched fluid.

The first pass target cell enriched or target cell depleted fluid may be recycled and reintroduced into the biofluid or into the pretreated biofluid to flow through the microfluidic separation channel as a blend.

The method may further comprise dosing the at least one primary stream with a reagent to produce a dosed suspension. The at least one primary stream may be a target cell enriched fluid. The reagent may be selected from an antigen or activation reagent configured to biochemically induce cell activation. The biochemically induced activation may allow for selection of subclasses of types of cells, for instance lymphocytes or T cells, by exploiting the morphological changes of activated cells. In some instances, activated cells may be larger than non-activated cells and cell size may vary throughout the cell cycle. The difference in size may allow for differential separation with acoustic energy.

The method may further comprise flowing the target cell enriched fluid through a second microfluidic separation channel or through microfluidic separation channels arranged in series and applying acoustic energy to each separation channel. The dosed suspension may allow for selection of target cells at a certain stage of the cell cycle.

For instance, in some embodiments of the method, the target cells in the primary stream may be lymphocytes and the method may further comprise separating activated lymphocytes from non-activated lymphocytes in the primary stream. The method may further comprise dosing the lymphocyte enriched fluid with a reagent to produce the dosed suspension, flowing the dosed suspension into an inlet of a second microfluidic separation channel, and applying acoustic energy to the second microfluidic separation channel. Activated lymphocytes may accumulate within at least one primary stream along the second separation channel and non-activated lymphocytes may accumulate within at least one secondary stream along the second separation channel.

In certain embodiments the additive is capable of altering or regulating at least one of density of the biofluid, density of the target cells, density of the non-target cells, and the predetermined volume of the additive is determined to alter or regulate the biofluid to have a desired density or concentration of target cells or non-target cells. For instance, the predetermined volume of the additive may be determined to allow target cells or non-target cells to approach neutral acoustic buoyancy in the biofluid. In some embodiments, the predetermined volume of the additive is determined to alter or regulate the density of the biofluid to a density of between about 1.00 g/mL and about 1.15 g/mL or to density ranges or values within this range, as previously discussed.

In some embodiments, the additive is capable of altering or regulating at least one of HCT% of the biofluid, concentration of the target cells, or concentration of the non-target cells, and the predetermined volume of the additive is determined to alter or regulate the HCT% of the biofluid to be less than about 10%. For instance, the predetermined volume of the additive may be determined to alter or regulate the HCT% of the biofluid to be less than about 30%, less than about 25%, less than about 20%, less than about 15%, less than about 10%, or less than about 5%.

The function and advantages of the invention can be further understood from the description of the figures below, which further illustrate the benefits and/or advantages of the one or more systems and techniques of the invention but do not exemplify the full scope of the invention.

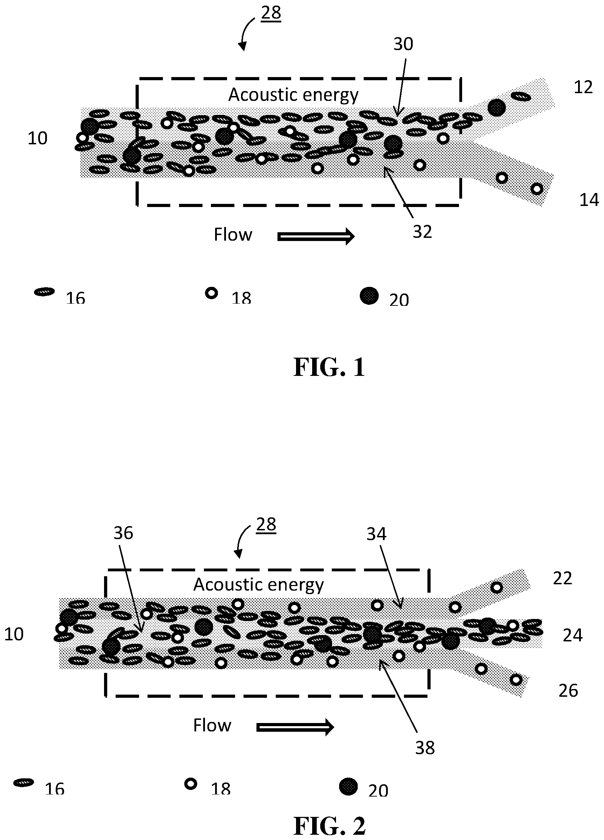

As shown in the exemplary concept schematic drawing of FIG. 1, a biofluid comprising target cells 18 and non-target cells 16 and 20 is flowed through microfluidic separation channel 28, through the inlet 10. Acoustic energy is applied to the separation channel 28 within the illustrated dotted line rectangle. Acoustic energy may be applied by attaching a piezoelectric transducer (not shown) to one wall of the separation channel. Target cells 18 accumulate within primary stream 32 and exit the separation channel 28 through first outlet 14. Target cell enriched fluid exits the first outlet 14. Non-target cells 16 and 20 accumulate within secondary stream 30 and exit the separation channel through second outlet 12. The non-target cells 16 and 20 are contained in a waste fluid. In some embodiments, the target cell enriched fluid within the primary stream 32 is collected.

Similarly, as shown in the exemplary concept schematic drawing of FIG. 2, the biofluid comprising target cells 18 and non-target cells 16 and 20 is flowed through the microfluidic separation channel 28 through inlet 10. In the embodiment exemplified in FIG. 2, target cells 18 essentially accumulate within two primary streams, 34 and 38, at the periphery of the separation channel 28, upon being subjected to the acoustic energy. Non-target cells 16 and 20 essentially accumulate within the central secondary stream 36. The primary streams 34 and 38 (target cell enriched fluid) exit the separation channel 28 through peripheral first outlets 22 and 26, while the secondary stream 36 (waste fluid) exits the separation channel 28 through second outlet 24. In this exemplary embodiment, non-target cells 16 and 20 are more susceptible to the acoustic energy, so they travel rapidly to the central region (secondary stream 36) of the separation channel 28, while the target cells 18 experience a weaker force from the acoustic energy and remain in the peripheral region of the separation channel 28 (primary streams 34 and 38).

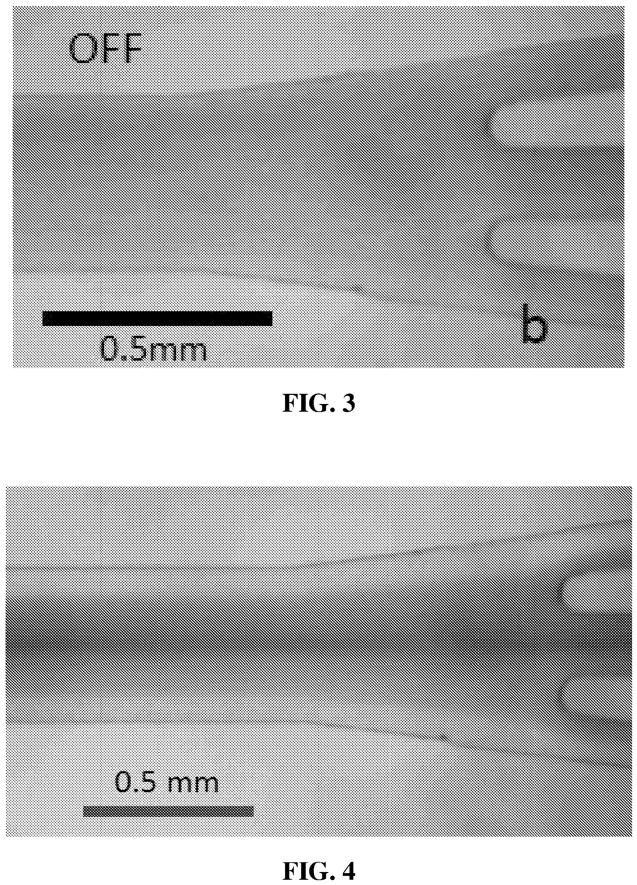

FIG. 3 and FIG. 4 are microscopic images of the downstream end of a microfluidic separation channel. In FIG. 3, the microfluidic separation channel is receiving no acoustic energy. As shown in the image, a homogeneous cell suspension is flowing through the channel with no separation. In FIG. 4, the microfluidic separation channel is receiving acoustic energy. Non-target cells, shown as the darker shade, can be seen traveling through the center stream, while target cells (not individually visible in the images) travel through the outer streams. The separation as seen in FIG. 4 is much greater than that seen in FIG. 3.

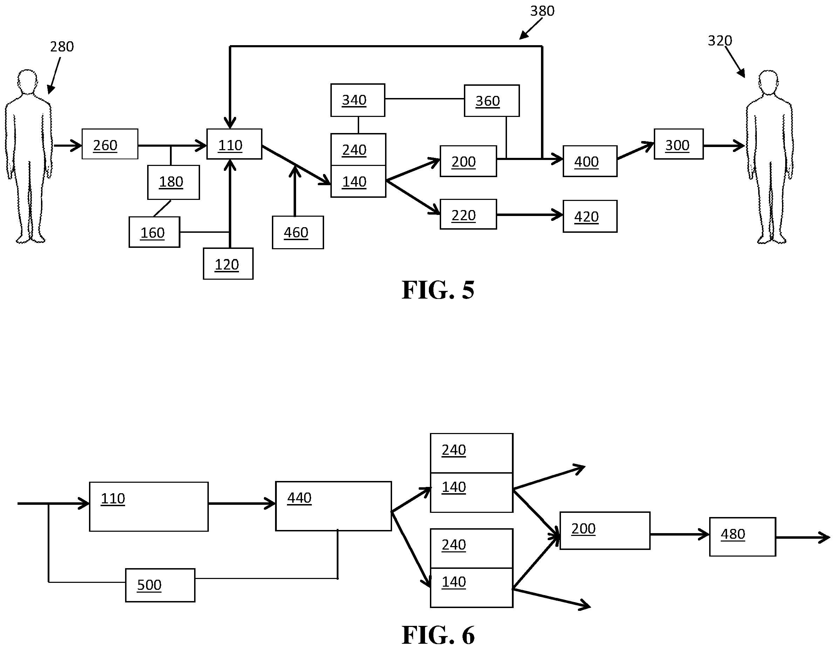

As shown in FIG. 5 (not according to the present invention), there is provided a system for microfluidic separation of target cells and non-target cells in a biofluid may comprise a source of a biofluid 110, a source of an additive 120, and a microfluidic separation channel 140 coupled to an acoustic transducer 240. The system may further comprise a sensor 180 configured to measure a parameter of an input biofluid and a sensor 360 configured to measure a parameter of a primary stream. The sensors may be electrically connected to control modules 340 and 160, such that control module 340 is configured to alter or regulate an input parameter of the acoustic transducer 240 and the control module 160 is configured to introduce a predetermined volume of the additive into the biofluid.

The system may further comprise intraluminal line 260 fluidly connected to donor subject 280 and second intraluminal line 300 fluidly connected to recipient subject 320. Recipient subject 320 and donor subject 280 may be the same subject. The microfluidic separation channel 140 may separate pretreated biofluid into a primary stream and a secondary stream, such that the primary stream comprising target cells (target cell enriched fluid) is directed to primary stream collection channel 220 and the secondary stream comprising non-target cells (target cell depleted fluid) is directed to secondary stream collection channel 220. The primary stream may be recycled back to the source of the biofluid 110 through recycle line 380 or may be post-treated in post-treatment chamber 400. In some embodiments, the post-treatment chamber 400 is fluidly connected to the intraluminal line 300. The secondary stream may be collected in collection vessel 420. The system may further comprise a source of a second fluid 460 fluidly connected to the microfluidic separation channel 140. Turning to FIG. 6, the system for microfluidic separation of target cells and non-target cells in a biofluid may further comprise two or more microfluidic separation channels 140. In the embodiment as shown, each microfluidic separation channel 140 is coupled to an acoustic transducer 240, however the system may comprise one acoustic transducer 240 coupled to more than one microfluidic separation channel 140. The two or more microfluidic separation channels 140 may be fluidly connected to a manifold 440, which may be fluidly or electrically connected to a sensor 500. The manifold 440 may be configured distribute the biofluid to the microfluidic separation channels 140 in response to a measurement received from the sensor 500 of an input biofluid load upstream of the biofluid source 110. In some embodiments, the system comprises a collection channel 200 downstream from the microfluidic separation channels 140 configured to collect the primary stream from the microfluidic separation channels 140. The system may further comprise a collection vessel 480 downstream from the collection channel 200.

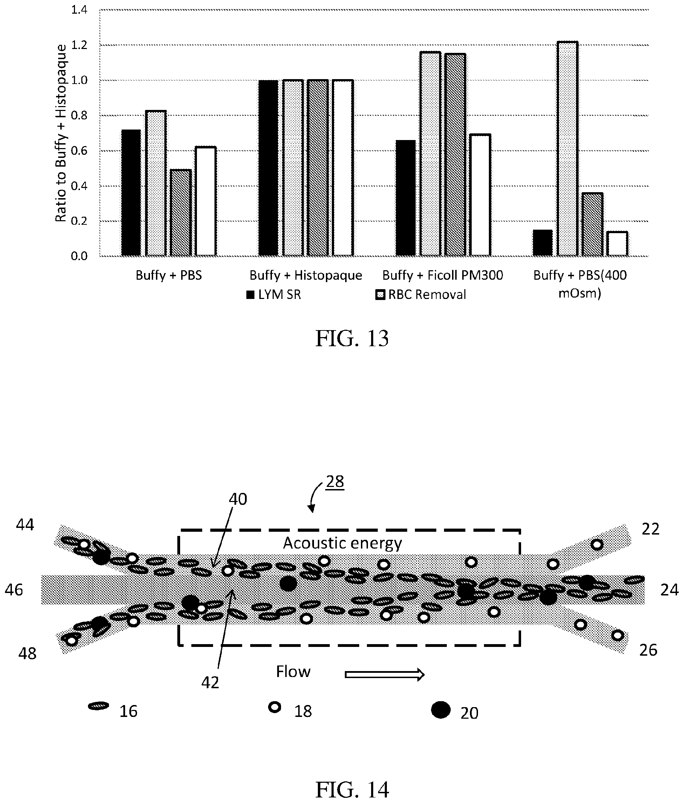

As shown in exemplary concept schematic drawing of FIG. 14, a second fluid 42 may be flowed through the microfluidic separation channel 28 with pretreated biofluid 40, in essentially parallel flow. The second fluid 42 enters the microfluidic separation channel 28 through central inlet 46, while pretreated biofluid 40 enters the microfluidic separation channel 28 through peripheral inlets 44 and 48. The second fluid 42 does not comprise cells as it enters the separation channel 28. Non-target cells 16 and 20 are driven towards the center stream by the applied acoustic energy, and exit the separation channel through waste outlet 24. Target cells 18 are essentially buoyant within the microfluidic separation channel 28, and are not driven to the central stream. The estimated recovery in the exemplary embodiment of FIG. 14 is calculated to be about 70%. Comparatively, the estimated recovery in an embodiment without introducing a second fluid, such as the one exemplified in FIG. 2, is about 65%.

**Examples:**

**Example 1: Acoustic Separation for Purification of Lymphocytes**

Separation of lymphocytes from human buffy coat product was performed with a microfluidic separation channel. The buffy coat was separated and collected from human whole blood. Buffy coat was flowed through a microfluidic separation channel at a residence time of about 1 second, and ultrasonic waves were applied to the channel to oscillate a portion of the channel having a cross section on the scale of the ultrasonic wavelength (~1mm). The acoustic energy on the channel was applied to drive cells toward an axial center stream.

Lymphocytes experienced a weaker force than erythrocytes and other leukocytes, due to the difference between a lymphocyte's size and density as compared to the alternate cells. As the blood buffy coat was flowed through the channel and subjected to the acoustic energy, the lymphocyte population accumulated along primary streams at the outside of the channel, the lymphocyte enriched fluid was separated by a branching in the channel, for instance such as the one shown in FIG. 4. The lymphocyte enriched fluid was collected and analyzed.

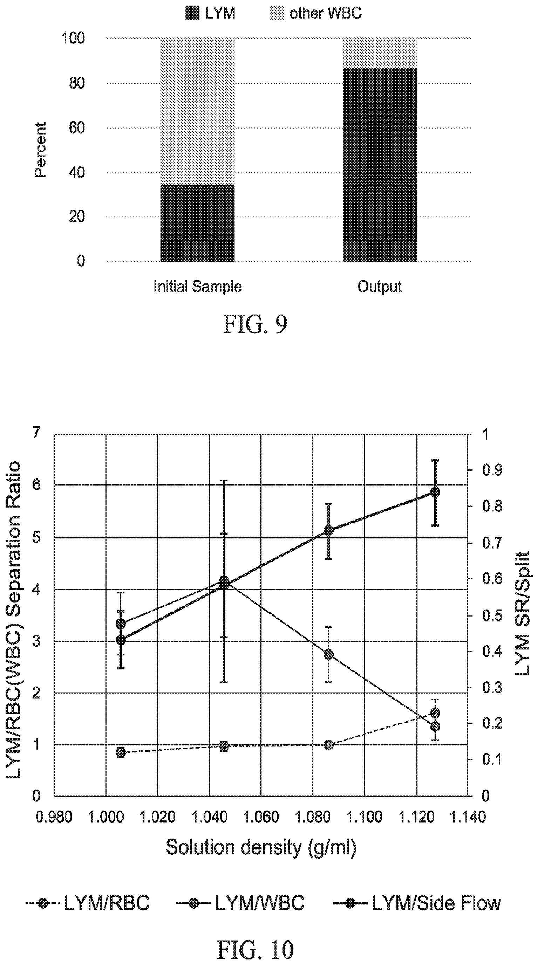

Initial output cell counts were measured with a standard hematology analyzer. The results are shown in the graph of FIG. 9. In the lymphocyte enriched output suspension, lymphocyte population, as compared to other leukocytes, was enriched from 34% (initial population) to 87% (output population). Lymphocytes were enriched 2.5X by single pass through the microfluidic separation channel. Total lymphocyte recover was 21%. Erythrocyte concentration was reduced by 50%. Lymphocyte recovery and separation from erythrocytes can be increased with additives, by device tuning (e.g. tuning the input and/or output parameters of the acoustic transducer), and by performing repeat passes through the separation channel.

Generally, without wishing to be bound to a particular theory, it is believed that non-target cells are focused toward the center stream, while lymphocytes are weakly focused toward the center stream, allowing for retention in peripheral streams.

Accordingly, lymphocytes can be selectively separated from like cells (leukocytes) with the systems and methods described herein.

**Example 2: Acoustic Separation with Density Gradient Medium**

Blood buffy coat comprising lymphocytes and non-target cells was subjected to acoustic energy, generally as described above. Prior to flowing the buffy coat through a microfluidic separation channel, buffy coat samples were pretreated by diluting with a density gradient medium at diluent densities ranging between about 1.00 and 1.15 (g/mL). The results are measured in separation ratio, a quantitative measurement of the ratio of cells in the product (separation efficiency).

The separation Ratio for any subpopulation x, where "side" is the primary stream and "center" is the secondary stream.

$$SR_x = \frac{n_{x,\,side}}{n_{x,side} + n_{x,\,center}}$$

The fraction of the stream out the side channel (primary stream), also referred to as the flow split:

$$FR_{side} = \frac{V_{side}}{V_{center}}$$

The separation ratio results biofluid diluted with density gradient medium are summarized in the graph shown in FIG. 10. As shown, the density gradient medium provides efficient separation of lymphocytes from other leukocytes, but does not affect the separation of lymphocytes from erythrocytes, which remains constant around 1.0 for increasing solution density. A maximum separation of lymphocytes from other leukocytes is effectuated near the density of the lymphocytes (approximately 1.06 g/mL). Without wishing to be bound to a particular theory, it is believed the density gradient medium does not efficiently affect separation of cells from erythrocytes in this range because the density of the erythrocytes remains significantly higher than that of the suspending fluid.

Accordingly, lymphocyte separation from leukocytes in a biofluid can be performed with superior results by pretreating the biofluid with an additive, such as a density gradient medium.

Without wishing to be bound to a particular theory, it is believed cell separation by pretreatment with additives capable of altering density of the biofluid, density of the target cells, density of the non-target cells, size of the target cells, size of the non-target cells, compressibility of the biofluid, compressibility of the target cells, compressibility of the non-target cells, aggregation potential of the target cells, and aggregation potential of the non-target cells will provide superior results over no pretreatment because the rate at which the cells migrate generally depends on cell size, density, and compressibility relative to the density and compressibility of the suspending biofluid.

**Example 3: Acoustic Separation with Cell Aggregator**

Blood buffy coat comprising lymphocytes and non-target cells was subjected to acoustic energy, generally as described above. Prior to flowing the buffy coat through a microfluidic separation channel, buffy coat samples were pretreated by introducing Ficoll™ PM 300 cell media (GE Healthcare, Chicago, IL), a long-chain polysaccharide was introduced into the biofluid. The samples pretreated with Ficoll™ were compared to samples pretreated Histopaque® media (distributed by Sigma-Aldrich, St. Louis, MO).

The results are summarized in the graph of FIG. 13. The samples pretreated with Ficoll™ exhibited better erythrocyte removal than the samples pretreated with Histopaque® However, the Ficoll™ samples exhibited lower lymphocyte recovery than the Histopaque® samples. Nonetheless, both samples exhibited improved erythrocyte removal and lymphocyte recovery than the control sample pretreated with PBS alone.

Accordingly, pretreating the biofluid with Ficoll™ provides superior non-target cell removal, but inferior target cell recovery, than pretreating the biofluid with Histopaque®. Furthermore, lymphocyte separation from leukocytes in a biofluid can be performed with superior results by pretreating the biofluid with Ficoll™ or Histopaque® as compared to pretreating the biofluid with PBS alone.

Accordingly, pretreating the biofluid with a cell aggregator provides superior non-target cell removal, but inferior target cell recovery, than pretreating the biofluid with a density gradient medium. Furthermore, lymphocyte separation from leukocytes in a biofluid can be performed.

**Example 4: Acoustic Separation with Density Gradient Medium and Cell Aggregator**

Blood buffy coat comprising lymphocytes and non-target cells was subjected to acoustic energy, generally as described above. Prior to flowing the buffy coat through a microfluidic separation channel, buffy coat samples were pretreated by diluting with an additive comprising a density gradient medium and cell aggregator to provide a solution density ranging between about 1.00 and 1.10 (g/mL). Specifically, the samples were pretreated with Histopaque® media (Sigma-Aldrich, St. Louis, MO).

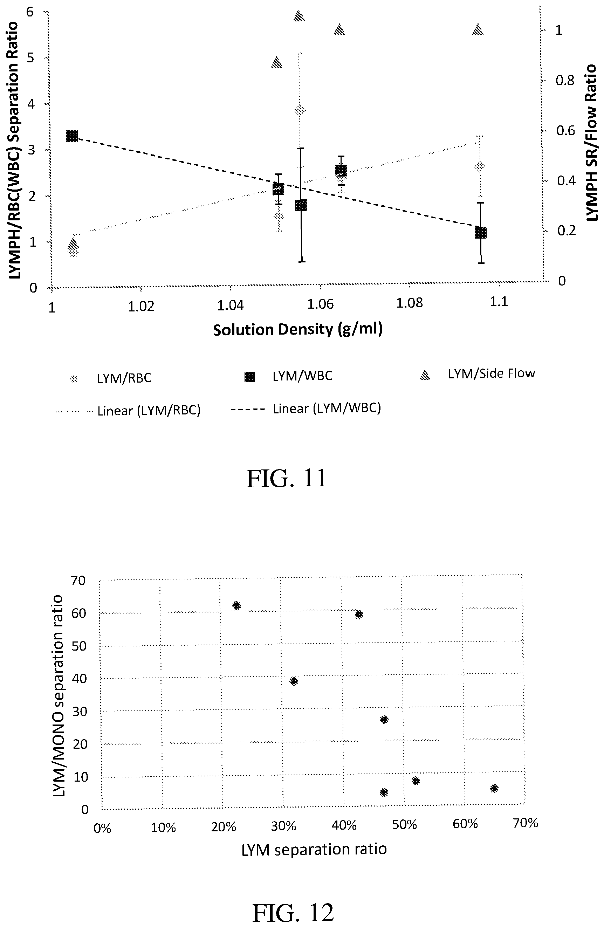

The results are summarized in the graph of FIG. 11. The graph shows a much better enrichment of lymphocytes from erythrocytes, as compared to the density gradient medium alone (FIG. 10). The lymphocyte/erythrocyte trendline shows that the additive comprising a density gradient medium and a cell aggregator displays a generally increasing separation of lymphocytes from erythrocytes with increasing solution density. In contrast, the lymphocyte/leukocyte trendline displays a generally decreasing separation of lymphocytes from leukocytes with increasing solution density. The optimal solution density is the point at which the trendlines cross, or near 1.06 g/mL, which is the approximate density of the lymphocytes. Accordingly, lymphocytes can be separated from erythrocytes and leukocytes most efficiently with an additive configured to alter the solution density to about 1.06 g/mL.

Furthermore, lymphocyte separation from erythrocytes and leukocytes in a biofluid is more efficient when the biofluid is pretreated with both a density gradient medium and a cell aggregator. The data show there is a synergistic result for the combination of mediums when compared to cell separation with each medium alone.

## Conclusion - Comparative Results

In similar lymphocyte separation experiments, biofluid diluted with a long-chain polysaccharide (cell aggregator) and PBS experienced better erythrocyte removal, but lower lymphocyte recovery than biofluid samples diluted in a density gradient medium and a cell aggregator. Biofluid diluted with high salt PBS (400 mOsm PBS) experienced better erythrocyte removal, but lower lymphocyte recovery when compared to biofluid diluted in the density gradient medium and cell aggregator. Finally, biofluid diluted in isotonic PBS experienced decreased performance across all metrics, when compared to biofluid diluted in the density gradient medium and cell aggregator, and generally as compared to the other experimental samples.

Accordingly, pretreating biofluid with an additive may result in increased separation between target cells and non-target cells, as compared to acoustic separation of biofluid alone.

## Example 5: Comparison between Target Cells - Lymphocyte and Monocyte Separation Ratio

Biofluid comprising lymphocytes and monocytes was flowed through a microfluidic separation channel and subjected to acoustic energy. The lymphocyte separation ratio was calculated as previously discussed. The monocyte separation ratio was compared to the lymphocyte separation ratio. The results are shown in the graph of FIG. 12. The data suggest that there is a differential separation between monocytes and lymphocytes. The results are significant because other separation processes, for example centrifugation, do not separate lymphocytes from monocytes. Accordingly, systems and methods disclosed herein allow for differential separation between cell types, including between different classes of leukocytes.

## Example 6: Comparison between Biofluids - Lymphocyte Purity and Recovery from Leukapheresis Product, Buffy Coat, and Whole Blood

Acoustic separation of lymphocytes from biofluid samples was performed, generally as described above. The biofluid samples included leukapheresis product, blood buffy coat, and whole blood. Generally, leukapheresis product comprises the highest ratio of leukocytes to other cells, blood buffy coat comprises a midrange ratio of leukocytes to other cells, and whole blood comprises the lowest ratio of leukocytes to other cells. Accordingly, as expected, lymphocyte recovery (percentage of lymphocyte in product to lymphocyte in biofluid sample), and lymphocyte purity (as a fraction of lymphocyte to total leukocyte concentration) is greatest when the biofluid is selected to be leukophoresis product, of the three example biofluids.

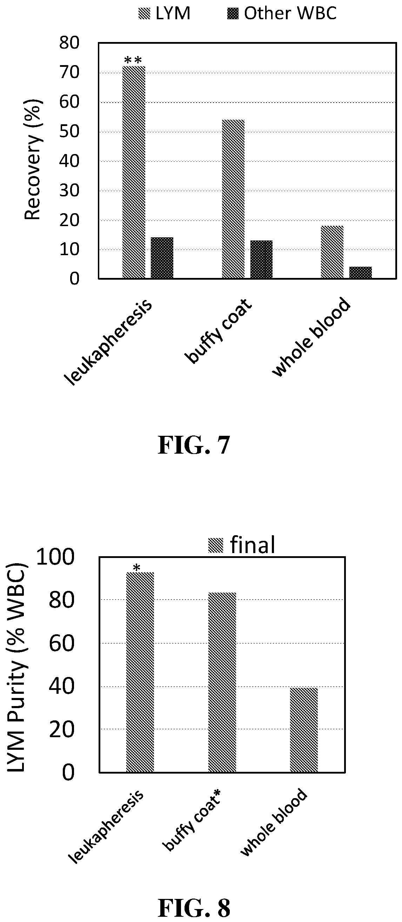

As shown in the results presented in the graphs of FIGS. 7 and 8, lymphocyte recovery from leukophoresis product, buffy coat, and whole blood is 71%, 54%, and 18%, respectively. Lymphocyte purity in these samples was high, at 93%, 83%, and 39%, respectively. Furthermore, the separation provided erythrocyte reduction (percentage of erythrocyte reduced from the biofluid sample) of about 94%, depending on the recovery goal. Accordingly, systems and methods for cell separation, as disclosed herein, may effectively recover and purify biofluid samples of various purities with a first pass acoustic separation process.

Those skilled in the art should appreciate that the parameters and configurations described herein are exemplary and that actual parameters and/or configurations will depend on the specific application in which the disclosed methods and materials are used. Those skilled in the art should also recognize or be able to ascertain, using no more than routine experimentation, equivalents to the specific embodiments disclosed. For example, those skilled in the art may recognize that the method, and components thereof, according to the present disclosure may further comprise a network or systems or be a component of a system for microfluidic cell separation. It is therefore to be understood that the embodiments described herein are presented by way of example only and that, within the scope of the appended claims and equivalents thereto; the disclosed embodiments may be practiced otherwise than as specifically described. The steps of the methods disclosed herein may be performed in the order illustrated or in alternate orders and the methods may include additional or alternative acts or may be performed with one or more of the illustrated acts omitted.

Further, it is to be appreciated that various alterations, modifications, and improvements will readily occur to those skilled in the art. Such alterations, modifications, and improvements are intended to be part of this disclosure. In other instances, an existing facility may be modified to utilize or incorporate any one or more aspects of the methods and systems described herein. Thus, in some instances, the systems may involve microfluidic cell separation. Accordingly the foregoing description and figures are by way of example only. Further the depictions in the figures do not limit the disclosures to the particularly illustrated representations.

The phraseology and terminology used herein is for the purpose of description and should not be regarded as limiting. As used herein, the term "plurality" refers to two or more items or components. The terms "comprising," "including," "carrying," "having," "containing," and "involving," whether in the written description or the claims and the like, are open-ended terms, i.e., to mean "including but not limited to." Thus, the use of such terms is meant to encompass the items listed thereafter, and equivalents thereof, as well as additional items. Only the transitional phrases "consisting of' aand "consisting essentially of," are closed or semi-closed transitional phrases, respectively, with respect to the claims. Use of ordinal terms such as "first," "second," "third," and the like in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

## Claims

1. An in vitro method of separating target cells (18) from non-target cells (16, 20) in a biofluid, comprising:

(a) altering at least one of the size of the target cells (18), size of the non-target cells (16, 20), compressibility of the biofluid, compressibility of the target cells (18), compressibility of the non-target cells (16, 20) and aggregation potential of the non-target cells (16,20); and altering at least one of density of the biofluid, density of the target cells, and density of the non-target cells by introducing a predetermined volume of at least one additive to the biofluid to produce a pre-treated biofluid, the predetermined volume of the at least one additive being selected to alter or regulate the biofluid to have a desired density;
(b) flowing the pre-treated biofluid into an inlet (10) of a microfluidic separation channel (28);
(c) applying acoustic energy to the pre-treated biofluid within the microfluidic separation channel (28) to separate the target cells (18) from the non-target cells (16, 20), such that the target cells (18) accumulate within at least one primary stream (32) along the separation channel (28) and the non-target cells (16, 20) accumulate within at least one secondary stream (30) along the separation channel (28);
(d) collecting the at least one primary stream (32) comprising the target cells (18); and
(e) optionally separately collecting the at least one secondary stream (30) comprising the non-target cells (16, 20).

2. The method of claim 1, further comprising selecting the at least one additive from the group consisting of a cell aggregator, deionized water, a detergent, a surfactant, a solution to regulate salinity of the biofluid, a solution to regulate tonicity of the biofluid, a solution to regulate viscosity of the biofluid, a solution to regulate osmolality of the biofluid, a solution to regulate ion concentration of the biofluid, and combinations thereof.

3. The method of claim 2, further comprising:

(a) selecting the cell aggregator to be a platelet activator comprising thrombin, type II collagen, adenosine diphosphate, or ristocetin;
(b) wherein the cell aggregator comprises long-chain polysaccharide, optionally having a molecular weight between 100 and 500 kD; optionally a long-chain polysaccharide is present at a concentration of between 0.5% (w/v) and 25% (w/v); or
(c) selecting the cell aggregator to be a solution comprising antibodies that bind and aggregate non-target cells (16, 20).

4. The method of claim 1 comprising introducing at least two additives, comprising a first additive to alter at least one of the size of the target cells (18), size of the non-target cells (16, 20), compressibility of the biofluid, compressibility of the target cells (18), compressibility of the non-target cells (16, 20), and aggregation potential of the non-target cells (16, 20); and a second additive to alter at least one of density of the biofluid, density of the target cells (18), and density of the non-target cells (16, 20).

5. The method of claim 4, comprising selecting the second additive from the group consisting of a density gradient medium, a density additive, and combinations thereof; optionally comprising selecting the density additive to be a nonionic iodinated compound and introducing the predetermined volume of the density additive to regulate the density of the biofluid to a density of between 1.00 g/mL and 1.15 g/mL; optionally to regulate the density of the biofluid to a density of between 1.04 g/mL to 1.07 g/mL.

6. The method of claim 1, further comprising:

(a) selecting the biofluid from blood buffy coat, leukapheresis product, peripheral blood, whole blood, lymph fluid, synovial fluid, spinal fluid, bone marrow, ascities fluid, and combinations or subcomponents thereof; or
(b) selecting the target cells (18) to be leukocytes selected from the group consisting of mononuclear cells, lymphocytes, monocytes, granulocytes, agranulocytes, macrophages, T cells, B cells, NK cells, subclasses thereof, and combinations thereof.

7. The method of claim 1, further comprising dosing the at least one primary stream (32) with a reagent to produce a dosed suspension, the reagent selected from an antigen or activation reagent configured to biochemically induce cell activation; and selecting the target cells (18) to be lymphocytes and separating activated lymphocytes from non-activated lymphocytes in the dosed suspension.

8. The method of claim 7, wherein selecting the target cells (18) to be lymphocytes and separating activated lymphocytes from non-activated lymphocytes in the dosed suspension comprises:

(a) flowing the dosed suspension into an inlet (10) of a second microfluidic separation channel (28); and
(b) applying acoustic energy to the second microfluidic separation channel (28), such that the activated lymphocytes accumulate within at least one primary stream (32) along the second separation channel (28) and the non-activated lymphocytes accumulate within at least one secondary stream (30) along the second separation channel (28).

9. The method of claim 1, further comprising at least one of:

(a) flowing a second fluid (42) adjacent to the biofluid (40) into an inlet (46) of the microfluidic separation channel (28), such that the biofluid (40) and the second fluid (42) flow in substantially parallel, substantially laminar flow; or
(b) flowing the pre-treated biofluid (40) into the inlet (44, 48) of the microfluidic separation channel (28) at a flow rate of between 0.03 mL/min to 0.5 mL/min.

10. The method of claim 1, comprising introducing the at least one additive to regulate the aggregation potential of the non-target cells, the non-target cells comprising erythrocytes; or platelets.

11. The method of claim 1, wherein the at least one additive is physiologically acceptable, and the method comprises introducing a safely injectable amount of the physiologically acceptable additive.

12. The method of claim 1, wherein the predetermined volume of the additive is selected to alter or regulate the biofluid to have a desired density, concentration of target cells (18), or concentration of non-target cells (16, 20), optionally wherein the predetermined volume of the additive is selected to allow the target cells (18) or the non-target cells (16, 20) to approach neutral acoustic buoyancy in the biofluid.

13. The method of claim 1, wherein the additive is further capable of altering or regulating at least one of haematocrit (HCT%) of the biofluid, concentration of target cells (18), or concentration of non-target cells (16, 20), and optionally wherein a predetermined volume of the at least one additive is selected to alter or regulate the HCT% of the biofluid to be less than about 10%.

14. The method of claim 1, further comprising measuring at least one of density of the biofluid, haematocrit (HCT%) of the biofluid, concentration of target cells (18) in the biofluid, and concentration of non-target cells (16, 20) in the biofluid, and selecting a predetermined volume of the at least one additive in response to the measurement.

15. The method of claim 1, wherein the biofluid is a blood sample or a blood product and the target cells are a class of leukocytes selected from the group consisting of lymphocytes, monocytes, granulocytes, agranulocytes, and macrophages; optionally wherein:

(a) the target cells are a class of lymphocytes selected from T cells, B cells, and NK cells, optionally a class of T cells selected from CD4+ cells, CD8+ cells, $T_H$ cells, $T_{CM}$, $T_{FH}$;
(b) the target cells are lymphocytes or a subclass thereof, and the non-target cells comprise erythrocytes; or

(c) the biofluid is leukapheresis product.

**Patentansprüche**

1. In-vitro-Verfahren zum Abtrennen von Zielzellen (18) von Nicht-Zielzellen (16, 20) in einem Biofluid, umfassend:

   (a) Ändern mindestens eines von der Größe der Zielzellen (18), der Größe der Nicht-Zielzellen (16, 20), der Kompressibilität des Biofluids, der Kompressibilität der Zielzellen (18), der Kompressibilität der Nicht-Zielzellen (16, 20) und dem Aggregationspotential der Nicht-Zielzellen (16, 20); und Ändern mindestens eines von der Dichte des Biofluids, der Dichte der Zielzellen und der Dichte der Nicht-Zielzellen durch Einbringen eines vorbestimmten Volumens mindestens eines Zusatzstoffs in das Biofluid, um ein vorbehandeltes Biofluid herzustellen, wobei das vorbestimmte Volumen des mindestens einen Zusatzstoffs ausgewählt ist, um das Biofluid so zu ändern oder zu regulieren, dass es eine gewünschte Dichte aufweist;
   (b) Strömenlassen des vorbehandelten Biofluids in einen Einlass (10) eines Mikrofluidik-Trennkanals (28);
   (c) Anlegen von akustischer Energie an das vorbehandelte Biofluid innerhalb des Mikrofluidik-Trennkanals (28), um die Zielzellen (18) von den Nicht-Zielzellen (16, 20) abzutrennen, so dass sich die Zielzellen (18) mindestens in einem Primärstrom (32) entlang des Trennkanals (28) ansammeln und sich die Nicht-Zielzellen (16, 20) mindestens in einem Sekundärstrom (30) entlang des Trennkanals (28) ansammeln;
   (d) Auffangen des mindestens einen Primärstroms (32), der die Zielzellen (18) umfasst; und
   (e) optional getrenntes Auffangen des mindestens einen Sekundärstroms (30), der die Nicht-Zielzellen (16, 20) umfasst.

2. Verfahren nach Anspruch 1, ferner umfassend ein Auswählen des mindestens einen Zusatzstoffs aus der Gruppe bestehend aus einem Zellaggregator, entionisiertem Wasser, einem Detergens, einem Tensid, einer Lösung zum Regulieren des Salzgehalts des Biofluids, einer Lösung zum Regulieren der Tonizität des Biofluids, einer Lösung zum Regulieren der Viskosität des Biofluids, einer Lösung zum Regulieren der Osmolalität des Biofluids, einer Lösung zum Regulieren der Ionenkonzentration des Biofluids und Kombinationen davon.

3. Verfahren nach Anspruch 2, ferner umfassend:

   (a) Auswählen des Zellaggregators als Thrombozytenaktivator, der Thrombin, Typ-II-Kollagen, Adenosindiphosphat oder Ristocetin umfasst;
(b) wobei der Zellaggregator langkettiges Polysaccharid umfasst, optional mit einem Molekulargewicht zwischen 100 und 500 kD; optional ist ein langkettiges Polysaccharid in einer Konzentration zwischen 0,5 % (Gew./Vol.) und 25 % (Gew./Vol.) vorhanden; oder
(c) Auswählen des Zellaggregators als eine Lösung, die Antikörper umfasst, die Nicht-Zielzellen binden und aggregieren (16, 20).

4. Verfahren nach Anspruch 1, umfassend ein Einbringen von mindestens zwei Zusatzstoffen, umfassend einen ersten Zusatzstoff, um mindestens eines von der Größe der Zielzellen (18), der Größe der Nicht-Zielzellen (16, 20), der Kompressibilität des Biofluids, der Kompressibilität der Zielzellen (18), der Kompressibilität der Nicht-Zielzellen (16, 20) und dem Aggregationspotential der Nicht-Zielzellen (16, 20) zu ändern; und einen zweiten Zusatzstoff, um mindestens eines von einer Dichte des Biofluids, einer Dichte der Zielzellen (18) und einer Dichte der Nicht-Zielzellen (16, 20) zu ändern.

5. Verfahren nach Anspruch 4, umfassend ein Auswählen des zweiten Zusatzstoffs aus der Gruppe bestehend aus einem Dichtegradientenmedium, einem Dichtezusatzstoff und Kombinationen davon; optional umfassend ein Auswählen des Dichtezusatzstoffs als nichtionische iodierte Verbindung und ein Einbringen des vorbestimmten Volumens des Dichtezusatzstoffs, um die Dichte des Biofluids auf eine Dichte zwischen 1,00 g/ml und 1,15 g/ml zu regulieren; optional, um die Dichte des Biofluids auf eine Dichte zwischen 1,04 g/ml bis 1,07 g/ml zu regulieren.

6. Verfahren nach Anspruch 1, ferner umfassend:

   (a) Auswählen der Bioflüssigkeit aus Buffy-Coat des Blutes, Leukaphereseprodukt, peripherem Blut, Vollblut, Lymphflüssigkeit, Synovialflüssigkeit, Rückenmarksflüssigkeit, Knochenmark, Aszitesflüssigkeit und Kombinationen oder Unterkomponenten davon; oder
   (b) Auswählen der Zielzellen (18) als Leukozyten, ausgewählt aus der Gruppe bestehend aus mononukleären Zellen, Lymphozyten, Monozyten, Granulozyten, Agranulozyten, Makrophagen, T-Zellen, B-Zellen, NK-Zellen, Unterklassen davon und Kombinationen davon.

7. Verfahren nach Anspruch 1, ferner umfassend ein Dosieren des mindestens einen Primärstroms (32) mit einem Reagens, um eine dosierte Suspension herzustellen, wobei das Reagens aus einem Antigen oder einem Aktivierungsreagenz ausgewählt ist, das dazu konfiguriert ist, eine Zellaktivierung biochemisch zu induzieren; und Auswählen der Zielzellen (18) als Lymphozyten und Abtrennen aktivierter Lymphozyten von nicht aktivierten Lymphozyten in der dosierten Suspension.

8. Verfahren nach Anspruch 7, wobei das Auswählen der Zielzellen (18) als Lymphozyten und das Abtrennen aktivierter Lymphozyten von nicht aktivierten Lymphozyten in der dosierten Suspension Folgendes umfasst:

   (a) Strömenlassen der dosierten Suspension in einen Einlass (10) eines zweiten Mikrofluidik-Trennkanals (28); und
   (b) Anlegen von akustischer Energie an den zweiten Mikrofluidik-Trennkanal (28), so dass sich die aktivierten Lymphozyten in mindestens einem Primärstrom (32) entlang des zweiten Trennkanals (28) ansammeln und sich die nicht aktivierten Lymphozyten in mindestens einem Sekundärstrom (30) entlang des zweiten Trennkanals (28) ansammeln.

9. Verfahren nach Anspruch 1, ferner umfassend mindestens eines von:

   (a) Strömenlassen eines zweiten Fluids (42), das dem Biofluid (40) benachbart ist, in einen Einlass (46) des Mikrofluidik-Trennkanals (28), so dass das Biofluid (40) und das zweite Fluid (42) im Wesentlichen parallel fließen, im Wesentlichen laminar fließen; oder
   (b) Strömenlassen des vorbehandelten Biofluids (40) in den Einlass (44, 48) des Mikrofluidik-Trennkanals (28) mit einer Flussrate zwischen 0,03 ml/min und 0,5 ml/min.

10. Verfahren nach Anspruch 1, umfassend ein Einbringen des mindestens einen Zusatzstoffs, um das Aggregationspotential der Nicht-Zielzellen zu regulieren, wobei die Nicht-Zielzellen Erythrozyten umfassen; oder Thrombozyten.

11. Verfahren nach Anspruch 1, wobei der mindestens eine Zusatzstoff physiologisch verträglich ist und das Verfahren ein Einbringen einer sicher injizierbaren Menge des physiologisch verträglichen Zusatzstoffs umfasst.

12. Verfahren nach Anspruch 1, wobei das vorbestimmte Volumen des Zusatzstoffs ausgewählt wird, um das Biofluid so zu ändern oder zu regulieren, dass es eine gewünschte Dichte, Konzentration von Zielzellen (18) oder Konzentration von Nicht-Zielzellen (16, 20) aufweist, wobei optional das vorbestimmte Volumen des Zusatzstoffs so ausgewählt ist, dass es den Zielzellen (18) oder den Nicht-Zielzellen (16, 20) ermöglicht, sich einem neutralen akustischen Auftrieb in dem Biofluid anzunähern.

13. Verfahren nach Anspruch 1, wobei der Zusatzstoff ferner in der Lage ist, mindestens eines von Hämatokrit (HCT-%) des Biofluids, Konzentration von Zielzellen (18) oder Konzentration von Nicht-Zielzellen (16, 20) zu ändern oder zu regulieren und wobei optional ein vorbestimmtes Volumen des mindestens einen Zusatzstoffs ausgewählt wird, um den HCT-% des Biofluids auf weniger als etwa 10 % zu ändern oder zu regulieren.

14. Verfahren nach Anspruch 1, ferner umfassend ein Messen mindestens eines von Dichte des Biofluids, Hämatokrit (HCT-%) des Biofluids, Konzentration von Zielzellen (18) in dem Biofluid und Konzentration von Nicht-Zielzellen (16, 20) in dem Biofluid, und Auswählen eines vorbestimmten Volumens des mindestens einen Zusatzstoffs als Reaktion auf die Messung.

15. Verfahren nach Anspruch 1, wobei das Biofluid eine Blutprobe oder ein Blutprodukt ist und die Zielzellen eine Klasse von Leukozyten sind, ausgewählt aus der Gruppe bestehend aus Lymphozyten, Monozyten, Granulozyten, Agranulozyten und Makrophagen; wobei optional:

    (a) die Zielzellen eine Klasse von Lymphozyten ausgewählt aus T-Zellen, B-Zellen und NK-Zellen, optional eine Klasse von T-Zellen ausgewählt aus CD4+-Zellen, CD8+-Zellen, $T_H$-Zellen, $T_{CM}$, $T_{FH}$ sind;
    (b) die Zielzellen Lymphozyten oder eine Unterklasse davon sind und die Nicht-Zielzellen Erythrozyten umfassen; oder
    (c) das Biofluid ein Leukaphereseprodukt ist.

**Revendications**

1. Procédé in vitro de séparation de cellules cibles (18) de cellules non cibles (16, 20) dans un biofluide, comprenant :

    (a) la modification d'au moins l'un de la taille des cellules cibles (18), de la taille des cellules non cibles (16, 20), de la compressibilité du biofluide, de la compressibilité des cellules cibles (18), de la compressibilité des cellules non cibles (16, 20) et du potentiel d'agrégation des cellules non cibles (16, 20) ; et la modification d'au moins l'une de la densité du biofluide, de la densité des cellules cibles et de la densité des cellules non cibles en introduisant un volume prédéterminé d'au moins un additif dans le biofluide pour produire un biofluide prétraité, le volume prédéterminé de l'au moins un additif étant sélectionné pour modifier ou réguler le biofluide afin d'avoir une densité souhaitée ;

(b) l'écoulement du biofluide prétraité dans une entrée (10) d'un canal de séparation microfluidique (28) ;

(c) l'application d'une énergie acoustique au biofluide prétraité dans le canal de séparation microfluidique (28) pour séparer les cellules cibles (18) des cellules non cibles (16, 20), de sorte que les cellules cibles (18) s'accumulent dans au moins un flux primaire (32) le long du canal de séparation (28) et les cellules non cibles (16, 20) s'accumulent dans au moins un flux secondaire (30) le long du canal de séparation (28) ;

(d) la collecte de l'au moins un flux primaire (32) comprenant les cellules cibles (18) ; et

(e) éventuellement la collecte séparée de l'au moins un flux secondaire (30) comprenant les cellules non cibles (16, 20) .

2. Procédé selon la revendication 1, comprenant en outre la sélection d'au moins un additif dans le groupe constitué d'un agrégateur cellulaire, d'eau déminéralisée, d'un détergent, d'un tensioactif, d'une solution pour réguler la salinité du biofluide, d'une solution pour réguler la tonicité du biofluide, d'une solution pour réguler la viscosité du biofluide, d'une solution pour réguler l'osmolalité du biofluide, d'une solution pour réguler la concentration ionique du biofluide, et leurs combinaisons.

3. Procédé selon la revendication 2, comprenant en outre :

(a) la sélection de l'agrégateur cellulaire comme étant un activateur plaquettaire comprenant de la thrombine, du collagène de type II, de l'adénosine diphosphate ou de la ristocétine ;

(b) dans lequel l'agrégateur cellulaire comprend un polysaccharide à longue chaîne, ayant éventuellement un poids moléculaire compris entre 100 et 500 kD ; éventuellement un polysaccharide à longue chaîne est présent à une concentration comprise entre 0,5 % (p/v) et 25 % (p/v) ; ou

(c) la sélection de l'agrégateur cellulaire comme étant une solution comprenant des anticorps qui lient et agrègent des cellules non cibles (16, 20).

4. Procédé selon la revendication 1, comprenant l'introduction d'au moins deux additifs, comprenant un premier additif pour modifier au moins l'un de la taille des cellules cibles (18), de la taille des cellules non cibles (16, 20), de la compressibilité du biofluide, de la compressibilité des cellules cibles (18), de la compressibilité des cellules non cibles (16, 20) et du potentiel d'agrégation des cellules non cibles (16, 20) ; et un second additif pour modifier au moins l'une de la densité du biofluide, de la densité des cellules cibles (18) et de la densité des cellules non cibles (16, 20).

5. Procédé selon la revendication 4, comprenant la sélection du second additif dans le groupe constitué d'un milieu à gradient de densité, d'un additif de densité et de leurs combinaisons ; comprenant éventuellement la sélection de l'additif de densité comme étant un composé iodé non ionique et l'introduction du volume prédéterminé de l'additif de densité pour réguler la densité du biofluide à une densité comprise entre 1,00 g/ml et 1,15 g/ml ; éventuellement pour réguler la densité du biofluide à une densité comprise entre 1,04 g/ml à 1,07 g/ml.

6. Procédé selon la revendication 1, comprenant en outre :

(a) la sélection du biofluide à partir d'une couche leucocytaire sanguine, d'un produit de leucophérèse, de sang périphérique, de sang total, de liquide lymphatique, de liquide synovial, de liquide céphalo-rachidien, de moelle osseuse, de liquide d'ascite et de combinaisons ou de souscomposants de ceux-ci ; ou

(b) la sélection des cellules cibles (18) comme étant des leucocytes choisis dans le groupe constitué de cellules mononucléées, lymphocytes, monocytes, granulocytes, agranulocytes, macrophages, cellules T, cellules B, cellules NK, leurs sous-classes et leurs combinaisons.

7. Procédé selon la revendication 1, comprenant en outre le dosage de l'au moins un flux primaire (32) avec un réactif pour produire une suspension dosée, le réactif étant choisi parmi un antigène ou un réactif d'activation conçu pour induire biochimiquement l'activation cellulaire ; et la sélection des cellules cibles (18) pour être des lymphocytes et la séparation des lymphocytes activés des lymphocytes non activés dans la suspension dosée.

8. Procédé selon la revendication 7, dans lequel la sélection des cellules cibles (18) comme étant des lymphocytes et la séparation des lymphocytes activés des lymphocytes non activés dans la suspension dosée comprend :

(a) l'écoulement de la suspension dosée dans une entrée (10) d'un second canal de séparation microfluidique (28) ; et

(b) l'application d'une énergie acoustique au second canal de séparation microfluidique (28), de sorte que les lymphocytes activés s'accumulent dans au moins un flux primaire (32) le long du second canal de séparation (28) et les lymphocytes non activés s'accumulent dans au moins un flux secondaire (30) le long du second canal de séparation (28).

9. Procédé selon la revendication 1, comprenant en outre au moins l'un de :

> (a) l'écoulement d'un second fluide (42) adjacent au biofluide (40) dans une entrée (46) du canal de séparation microfluidique (28), de sorte que le biofluide (40) et le second fluide (42) s'écoulent selon un écoulement sensiblement parallèle, sensiblement laminaire ; ou
> (b) l'écoulement du biofluide prétraité (40) dans l'entrée (44, 48) du canal de séparation microfluidique (28) à un débit compris entre 0,03 ml/min et 0,5 ml/min.

10. Procédé selon la revendication 1, comprenant l'introduction de l'au moins un additif pour réguler le potentiel d'agrégation des cellules non cibles, les cellules non cibles comprenant des érythrocytes ; ou des plaquettes.

11. Procédé selon la revendication 1, dans lequel l'au moins un additif est physiologiquement acceptable, et le procédé comprend l'introduction d'une quantité injectable en toute sécurité de l'additif physiologiquement acceptable.

12. Procédé selon la revendication 1, dans lequel le volume prédéterminé de l'additif est choisi de manière à modifier ou réguler le biofluide pour avoir une densité, une concentration de cellules cibles (18) ou une concentration de cellules non cibles (16, 20) souhaitées, éventuellement dans lequel le volume prédéterminé de l'additif est choisi de manière à permettre aux cellules cibles (18) ou aux cellules non cibles (16, 20) d'approcher une flottabilité acoustique neutre dans le biofluide.

13. Procédé selon la revendication 1, dans lequel l'additif est en outre capable de modifier ou de réguler au moins l'un du taux d'hématocrite (HCT%) du biofluide, de la concentration de cellules cibles (18) ou de la concentration de cellules non cibles (16, 20), et éventuellement dans lequel un volume prédéterminé de l'au moins un additif est choisi de manière à modifier ou réguler le HCT% du biofluide pour qu'il soit inférieur à environ 10 %.

14. Procédé selon la revendication 1, comprenant en outre la mesure d'au moins un de la densité du biofluide, du taux d'hématocrite (HCT%) du biofluide, de la concentration de cellules cibles (18) dans le biofluide et de la concentration de cellules non cibles (16, 20) dans le biofluide, et la sélection d'un volume prédéterminé de l'au moins un additif en réponse à la mesure.

15. Procédé selon la revendication 1, dans lequel le biofluide est un échantillon de sang ou un produit sanguin et les cellules cibles sont une classe de leucocytes choisis dans le groupe constitué de lymphocytes, monocytes, granulocytes, agranulocytes et macrophages ; éventuellement dans lequel :

> (a) les cellules cibles sont une classe de lymphocytes choisis parmi les cellules T, les cellules B et les cellules NK, éventuellement une classe de cellules T choisies parmi les cellules CD4+, les cellules CD8+, les cellules $T_H$, $T_{CM}$, $T_{FH}$ ;
> (b) les cellules cibles sont des lymphocytes ou une sous-classe de ceux-ci, et les cellules non cibles comprennent des érythrocytes ; ou
> (c) le biofluide est un produit de leucophérèse.

**FIG. 1**

**FIG. 2**

**FIG. 3**

**FIG. 4**

**FIG. 5**

**FIG. 6**

EP 3 490 712 B1

**FIG. 7**

**FIG. 8**

FIG. 9

FIG. 10

FIG. 11

FIG. 12

FIG. 13

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016062975 A
- US 20160030660
- US 20160008532
- US 20130048565
- US 9504780 B